# EUROPEAN PATENT SPECIFICATION

(11) **EP 3 028 430 B1**
(45) Date of publication and mention of the grant of the patent: **20.06.2018**
(21) Application number: 14730433.1
(22) Date of filing: 06.05.2014
(51) Int. Cl.: H04L 29/06

(54) **SYSTEM ALLOWING ACCESS TO DEFINED ADDRESSE AFTER CHECK WITH ACCESS-LIST**
ANRICHTUNG ZUM ERLAUBEN VON ZUGRIFF AUF DEFINIERTE ADDRESSE HINSICHTLICH ZUGRIFFSLISTE
SYSTÈME POUR PERMETTRE L'ACCÈS A UNE ADRESSE BIEN-DEFINIE PAR RAPPORT A UNE LISTE D'ACCÈS

(30) Priority: 03.05.2013 GB 201308063
(43) Date of publication of application: 08.06.2016
(73) Proprietor: Rosberg System AS, 5542 Karmsund (NO)
(72) Inventor: ROSBERG, Odd Helge, NO 5542 Karmsund (NO)
(74) Representative: HGF Limited
(86) International application number: PCT/EP2014/059265
(87) International publication number: WO 2014/177725

(56) References cited:
- EP-A1- 2 372 971
- WO-A1-2008/049970
- WO-A1-2011/079836
- US-A1- 2006 085 528

## Description

This invention relates to an access control system.

### BACKGROUND

In homes and businesses there are a growing number of devices that require remote management, content delivery, data retrieval and other services. One example is metering of electricity and other utilities. Another example is home hospital solutions where medical readings are read securely into an external medical system. A further example is managing computers, printers, servers and connected home and office systems like security, refrigerators, cameras, dishwashers and multiple other devices.

Connecting these devices poses a huge threat in terms of security. There have already been examples of tampering with meter readings from power consumption meters to lower the electricity bill. More serious is the fact that one can disconnect and reconnect power remotely, and the fact that this may be done not only for one customer but for a whole area. Such problems have kept many suppliers from implementing such functionality or limiting this functionality significantly.

The present invention, at least in its preferred embodiments, seeks to provide a simple, scalable and profitable eco-system for infrastructure providers, such as telecommunications companies, to be able to accommodate a growing need to reach devices inside a home, a company and the in a simple and secured way and being able to charge the different operators for that access.

WO 2011/079836 discloses a method for controlling a call setup. US 2006/0085528 discloses a system and method for monitoring network communications for pestware.

### BRIEF SUMMARY OF THE DISCLOSURE

In accordance with the present invention there is provided an access control device as defined in claim 1.

Thus, in accordance with the invention the access control device facilitates data communication between remote service providers, such as power companies, and local devices, such as electricity meters. Local data communication may be via a local area network.

In this way, a single access control device can be used to provide data communication for multiple local devices and multiple service providers. Moreover, a particular service provider may be limited to data communication with only specified local devices.

The access control device may be configured to receive a connection request from the remote service provider. The connection request may include an identifier of the remote service provider. The access control device may be further configured to reject the connection request, and in response to the received connection request to compare the received identifier to a predefined list of identifiers for authorised remote service providers. If the identifier matches an identifier on the list, the access control device may be configured to identify a predefined address for the remote service provider, the address having been stored previously by the access control access control device, and to establish data communication with the predefined address. This provides a particularly secure access protocol that prevents unauthorised access.

In response to a successful connection request from a remote service provider, the access control device may be configured to establish data communication with a local device associated with the remote service provider.

The access control device may be configured to send a connection request to the local device, the connection request including an identifier of the access control device. The local device may be configured to reject the connection request, and in response to the received connection request to compare the received identifier to a predefined list of identifiers for authorised access control devices. If the identifier matches an identifier on the list, the local device may be configured to identify a predefined address for the access control device, the address having been stored previously by the local device, and to establish data communication with the predefined address. This ensures that a particular local device can only be accessed by a particular access control device.

The access control device may be configured to receive a connection request from the local device, the connection request including an identifier of the local device. The access control device may be further configured to reject the connection request, and in response to the received connection request to compare the received identifier to a predefined list of identifiers for authorised local devices. If the identifier matches an identifier on the list, the access control device may be configured to identify a predefined address for the local device, the address having been stored previously by the access control device, and to establish data communication with the predefined address. In response to a successful connection request from a local device, the access control device may be configured to establish data communication with a remote service provider associated with the local device.

The access control device may comprise a plurality of virtual machines, each configured to manage data communication between at least one respective remote service provider and at least one respective local device.

At least one local device may be a metering device, for example an electricity meter or water meter. At least one local device may be a medical sensor.

The invention extends to computer software which configures general-purpose data processing apparatus to operate as an access control access control device as described above.

### BRIEF DESCRIPTION OF THE DRAWINGS

Embodiments of the invention are further described hereinafter with reference to the accompanying drawings, in which:
Figure 1 is a schematic representation of an access control device according to an embodiment of the invention;
Figure 2 is a schematic representation of the operation of the access control device of Figure 1; and
Figure 3 is a further schematic representation of the operation of the access control device of Figure 1.

### DETAILED DESCRIPTION

Figures 1 to 3 show schematically an access control system for remote management of multiple devices according to an embodiment of the invention. As shown in Figure 1, the access control system is in the form of a gateway device comprising a plurality of virtual machines, VM1 ... VMN. The device comprises a secure administration space for configuring the operation of the virtual machines. The owner of the gateway device can connect to the gateway device via an owner interface in order to set access controls and the like. The term "virtual machine" (VM) as used herein indicates the software engine that controls connections, which may be any suitable means capable of performing the required functionality.

The owner of the gateway device may also be the local area network owner, i.e. a customer rather than a service provider or a telecommunications company. The secure administration space in the gateway device may be divided into several parts where one

Depending on the level of security required, alternative connection protocols may be used.

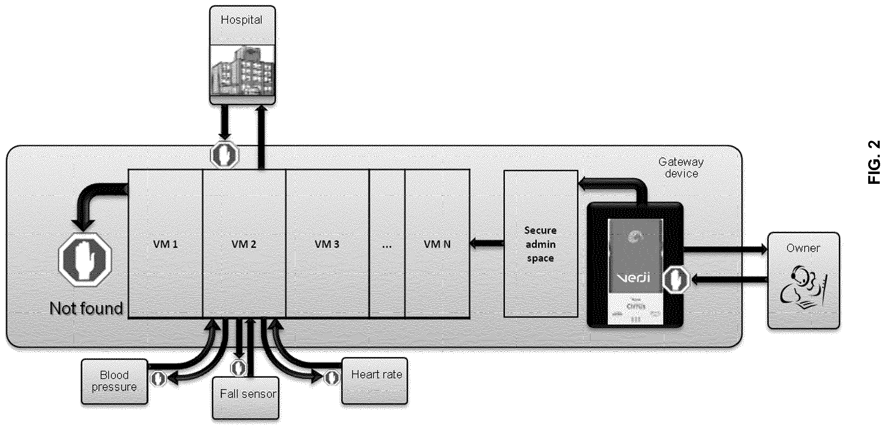

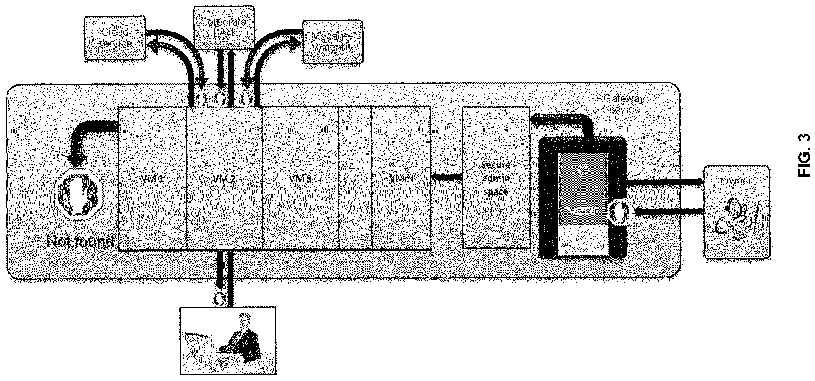

As shown in Figure 2, multiple local devices may communicate with a service provider via a virtual machine. In the example shown, a blood pressure monitor, a sensor indicating a patient has fallen and a heart rate monitor each communicate with a hospital. Similarly, as shown in Figure 3, multiple service providers may communicate with a single local device via a virtual machine. In the example shown, a user laptop communicates with a "cloud" data storage service, a corporate LAN and a management service provider.

The local devices may be connected to the gateway device via a local area network, which may be a corporate network, a home network, a personal area network, an in-car network, etc. The local area network may be wired, wireless or a combination of both. The gateway device provides secured access to the local devices in a standardized way while giving the owner of the gateway device complete control over the access rights to the local devices. In this way, instead of having one box for water metering, one for power metering, one for security systems and one for remote access to the network a single gateway device provides all of this secure access in a configurable manner.

The owner of the local devices and the gateway device sets the parameters of the access for different service providers. These include:
- Access rights to the local devices;
- Allowed service providers, identified by caller-ID (caller line identification), IP-address or other identifiers;
- Actions to perform, such as like starting a virtual machine, starting a virtual application, setting up communication, secured communication if needed, e.g. via VPN, credential checks and other security actions;
- A list of local devices or types of devices within the networked environment that a service provider is allowed to see;
- Creation and transfer of virtual machines, virtual applications, partial virtualization, para-virtualization and any other method of making a secured secluded environment for the different accesses in the gateway device.

The service provider may provide the local device, such as an electricity meter, within a customer's local area network. Access to the local device is then strictly regulated within the local device so that only the owner of the local device, e.g. the service provider, is allowed access to the setup of the device. The setup consists of deploying a table comprising:
- Allowed external identifiers, which may be any ID depending on the usage of the local device, for example caller-ID, IP address, Instant Message ID (using XMPP or similar technologies);
- An address to connect the service provider to a specific virtual machine if the callback protocol described above is used;
- Instructions on how to connect the service provider (callback, type of communication to use, which virtual machine to use, security checks, establishing a VPN connection or any other kind of command or set of commands).
- Allowed internal ID or ID's for the local device(s) the service provider wishes to contact within the customer's network, which may be any ID depending on the usage of the local device, for example caller-ID, IP address, Instant Message ID (using XMPP or similar technologies);
- An address to connect the local device to a specific virtual machine if the callback protocol described above is used;
- Instructions on how to connect the local device (callback, type of communication to use, which virtual machine to use, security checks, establishing a VPN connection or any other kind of command or set of commands);
- Instructions for scheduled connections to local devices for reporting back information to service providers for purposes such as metering, usage statistics, sensor instances or any other information relayed back to the service provider on a regular basis; and
- Event-based relay of information where the local device connects to the gateway device when certain events occurs, such as alarms, usage threshold limits being exceeded or any other event suited to trigger such an action.

The setup also includes deploying a VM or set of VM's using a virtual machine manager (VMM) or a hypervisor or similar technology to install one or a set of virtual machines that are completely isolated from each other. This may alternatively be done using virtual applications in a similarly isolated environment, a combination or using other methods of separating the different accesses from each other. These engines are then connected using the information in the table above.

The virtual machines are designed to only access a certain local device or certain local devices inside the customer's network. This is to prevent the service provider from accessing anything inside the customer's network beside the ones defined. This may be done in several ways, ranging from low level security solutions, such as MAC address range filtering, where a certain manufacturer has a certain range of MAC addresses to UUID (universally unique identifier) or more advanced methods using keys and tokens or other secure methods of identifying devices.

A local device can be set up to contact the specified VM in response to specified events, such as reaching a threshold value or when data needs to be delivered to the server. If the VM in the gateway device is not started, the hypervisor will start it to allow it to receive and relay the data to the correct service provider.

There may also be settings where a local device needs to talk to two or more of the VM's, an example of this may be a laptop that use one VM to communicate to a cloud service, another to communicate with the hospital and beside that has a connection to the internet using a default gateway. In this setting the local device either has an ID in each application, or an agent providing the intelligence needed or both.

In the same way one may also set up the gateway device to communicate with several local devices. One exemplification may be in a company where several multifunction devices communicate with one VM for servicing and usage reports.

The definition of an internal network may not only be a traditional network, but also a virtual network where devices are roaming, or several LAN's connected together. This may be achieved by traditional techniques such as VPN but also using mesh, instant messaging protocols (such as XMPP) or other similar technologies.

In summary, an access control system is configured for data communication with at least a first remote service provider via a wide area telecommunications network and for data communication with at least a first local device via local data communication. The access control system is configurable to facilitate data communication between the first remote service provider and the first local device.

Throughout the description and claims of this specification, the words "comprise" and "contain" and variations of them mean "including but not limited to", and they are not intended to (and do not) exclude other components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive.

## Claims

1. An access control device configured for data communication with at least a first remote service provider and a second remote service provider via a wide area telecommunications network and for data communication with at least a first local device and a second local device via local data communication,
wherein the access control device is configured to facilitate data communication between the first remote service provider and the first local device and to facilitate data communication between the second remote service provider and the second local device, said access control device being **characterised by** being configured to prevent data communication between the first remote service provider and the second local device and to prevent data communication between the second remote service provider and the first local device.

2. An access control device as claimed in claim 1, wherein the device comprises a plurality of virtual machines, each configured to manage data communication between at least one respective remote service provider and at least one respective local device.

3. An access control device as claimed in any preceding claim, wherein local data communication is via a local area network.

4. An access control device as claimed in any preceding claim, wherein at least one local device is a metering device.

5. An access control device as claimed in any preceding claim, wherein the access control device is configured to receive a connection request from one of the remote service providers, the connection request including an identifier of the remote service provider, to reject the connection request, and in response to the received connection request to compare the received identifier to a predefined list of identifiers for authorised remote service providers, and, if the identifier matches one of the identifiers on the list, to identify a predefined address for the remote service provider, the address having been stored previously by the access control device, and to establish data communication with the predefined address.

6. An access control device as claimed in any preceding claim, wherein in response to a successful connection request from one of the remote service providers, the access control device is configured to establish data communication with one of the local device associated with the remote service provider.

7. An access control device as claimed in any preceding claim, wherein the access control device is configured to send a connection request to one of the local devices, the connection request including an identifier of the access control device, the local device is configured to reject the connection request, and in response to the received connection request to compare the received identifier to a predefined list of identifiers for authorised access control devices, and, if the identifier matches one of the identifiers on the list, to identify a predefined address for the access control device, the address having been stored previously by the local device, and to establish data communication with the predefined address.

8. An access control device as claimed in any preceding claim, wherein the access control device is configured to receive a connection request from one of the local devices, the connection request including an identifier of the local device, the access control device is configured to reject the connection request, and in response to the received connection request to compare the received identifier to a predefined list of identifiers for authorised local devices, and, if the identifier matches one of the identifiers on the list, to identify a predefined address for the local device, the address having been stored previously by the access control device, and to establish data communication with the predefined address.

9. An access control device as claimed in any preceding claim, wherein in response to a successful connection request from one of the local devices, the access control device is configured to establish data communication with one of the remote service providers associated with the local device.

10. Computer software which configures general-purpose data processing apparatus to operate as an access control device as claimed in any preceding claim, the general-purpose data processing apparatus being configured to:
communicate data with at least a first remote service provider and a second remote service provider via a wide area telecommunications network;
communicate data with at least a first local device and a second local device via local data communication;
facilitate data communication between the first remote service provider and the first local device;
facilitate data communication between the second remote service provider and the second local device;
prevent data communication between the first remote service provider and the second local device; and
prevent data communication between the second remote service provider and the first local device.

## Patentansprüche

1. Zugriffssteuerungsvorrichtung, die für die Datenkommunikation mit wenigstens einem entfernten Serviceprovider und einem zweiten entfernten Serviceprovider über ein Wide Area-Telekommunikationsnetz und für die Datenkommunikation mit wenigstens einer ersten lokalen Vorrichtung und einer zweiten lokalen Vorrichtung über lokale Datenkommunikation gestaltet ist,
wobei die Zugriffssteuerungsvorrichtung so gestaltet ist, dass sie die Datenkommunikation zwischen dem ersten entfernten Serviceprovider und der ersten lokalen Vorrichtung ermöglicht und so dass sie die Datenkommunikation zwischen dem zweiten entfernten Serviceprovider und der zweiten lokalen Vorrichtung ermöglicht, wobei die Zugriffssteuerungsvorrichtung **dadurch gekennzeichnet ist, dass** sie so gestaltet ist, dass sie die Datenkommunikation zwischen dem ersten entfernten Serviceprovider und der zweiten lokalen Vorrichtung verhindert, und dass sie die Datenkommunikation zwischen dem zweiten entfernten Serviceprovider und der ersten lokalen Vorrichtung verhindert.

2. Zugriffssteuerungsvorrichtung nach Anspruch 1, wobei die Vorrichtung eine Mehrzahl virtueller Maschinen umfasst, die jeweils so gestaltet sind, dass sie die Datenkommunikation zwischen wenigstens einem entsprechenden entfernten Serviceprovider und wenigstens einer lokalen Vorrichtung managen.

3. Zugriffssteuerungsvorrichtung nach einem der vorstehenden Ansprüche, wobei die lokale Datenkommunikation über ein lokales Netzwerk erfolgt.

4. Zugriffssteuerungsvorrichtung nach einem der vorstehenden Ansprüche, wobei wenigstens eine lokale Vorrichtung eine Messvorrichtung ist.

5. Zugriffssteuerungsvorrichtung nach einem der vorstehenden Ansprüche, wobei die Zugriffssteuerungsvorrichtung so gestaltet ist, dass sie eine Verbindungsanforderung von einem der entfernten Serviceprovider empfängt, wobei die Verbindungsanforderung einen Bezeichner des entfernten Serviceproviders aufweist, um die Verbindungsanforderung abzuweisen, und um als Reaktion auf die empfangene Verbindungsanforderung den empfangenen Bezeichner mit einer vordefinierten Liste von Bezeichnern für autorisierte entfernte Serviceprovider zu vergleichen, und um, wenn der Bezeichner mit einem der Bezeichner auf der Liste übereinstimmt, eine vordefinierte Adresse für den entfernten Serviceprovider zu identifizieren, wobei die Adresse vorher durch die Zugriffssteuerungsvorrichtung gespeichert worden ist, und um die Datenkommunikation mit der vordefinierten Adresse herzustellen.

6. Zugriffssteuerungsvorrichtung nach einem der vorstehenden Ansprüche, wobei die Zugriffssteuerungsvorrichtung so gestaltet ist, dass sie als Reaktion auf eine erfolgreiche Verbindungsanforderung von einem der entfernten Serviceprovider die Datenkommunikation mit einer der lokalen Vorrichtungen herstellt, die dem entfernten Serviceprovider zugeordnet sind.

7. Zugriffssteuerungsvorrichtung nach einem der vorstehenden Ansprüche, wobei die Zugriffssteuerungsvorrichtung so gestaltet ist, dass sie eine Verbindungsanforderung an eine der lokalen Vorrichtungen sendet, wobei die Verbindungsanforderung einen Bezeichner der Zugriffssteuerungsvorrichtung aufweist, und um als Reaktion auf die empfangene Verbindungsanforderung den empfangenen Bezeichner mit einer vordefinierten Liste von Bezeichnern für autorisierte Zugriffssteuerungsvorrichtungen zu vergleichen, und um, wenn der Bezeichner mit einem der Bezeichner auf der Liste übereinstimmt, eine vordefinierte Adresse der Zugriffssteuerungsvorrichtung zu identifizieren, wobei die Adresse vorher durch die lokale Vorrichtung gespeichert worden ist, und um die Datenkommunikation mit der vordefinierten Adresse herzustellen.

8. Zugriffssteuerungsvorrichtung nach einem der vorstehenden Ansprüche, wobei die Zugriffssteuerungsvorrichtung so gestaltet ist, dass sie eine Verbindungsanforderung von einer der lokalen Vorrichtungen empfängt, wobei die Verbindungsanforderung einen Bezeichner der lokalen Vorrichtung aufweist, wobei die Zugriffssteuerungsvorrichtung so gestaltet ist, dass sie die Verbindungsanforderung abweist, und um als Reaktion auf die empfangene Verbindungsanforderung den empfangenen Bezeichner mit einer vordefinierten Liste von Bezeichnern für autorisierte lokale Vorrichtungen zu vergleichen, und um, wenn der Bezeichner mit einem der Bezeichner auf der Liste übereinstimmt, eine vordefinierte Adresse für die lokale Vorrichtung zu identifizieren, wobei die Adresse vorher durch die Zugriffssteuerungsvorrichtung gespeichert worden ist, und um die Datenkommunikation mit der vordefinierten Adresse herzustellen.

9. Zugriffssteuerungsvorrichtung nach einem der vorstehenden Ansprüche, wobei die Zugriffssteuerungsvorrichtung so gestaltet ist, dass sie als Reaktion auf eine erfolgreiche Verbindungsanforderung von einer der lokalen Vorrichtungen die Datenkommunikation mit einem der entfernten Serviceprovider herstellt, welcher der lokalen Vorrichtung zugeordnet ist.

10. Computersoftware, die eine Universaldatenverarbeitungsvorrichtung zu gestaltet, dass diese eine Zugriffssteuerungsvorrichtung nach einem der vorstehenden Ansprüche betreibt, wobei die Universaldatenverarbeitungsvorrichtung für folgende Zwecke gestaltet ist:
Kommunizieren von Daten mit wenigstens einem ersten entfernten Serviceprovider und einem zweiten entfernten Serviceprovider über ein Wide Area-Telekommunikationsnetz;
Kommunizieren von Daten mit wenigstens einer ersten lokalen Vorrichtung und einer zweiten lokalen Vorrichtung über eine lokale Datenkommunikation;
Ermöglichen der Datenkommunikation zwischen dem ersten entfernten Serviceprovider und der ersten lokalen Vorrichtung;
Ermöglichen der Datenkommunikation zwischen dem zweiten entfernten Serviceprovider und der zweiten lokalen Vorrichtung;
Verhindern der Datenkommunikation zwischen dem ersten entfernten Serviceprovider und der zweiten lokalen Vorrichtung; und
der Datenkommunikation zwischen dem zweiten entfernten Serviceprovider und der ersten lokalen Vorrichtung.

## Revendications

1. Dispositif de contrôle d'accès conçu pour la communication de données avec au moins un premier fournisseur de services à distance et un second fournisseur de services à distance par l'intermédiaire d'un réseau de télécommunications étendu et pour la communication de données avec au moins un premier dispositif local et un second dispositif local par l'intermédiaire d'une communication de données locale,
le dispositif de contrôle d'accès étant conçu pour faciliter la communication de données entre le premier fournisseur de services à distance et le premier dispositif local et pour faciliter la communication de données entre le second fournisseur de services à distance et le second dispositif local,
ledit dispositif de contrôle d'accès étant **caractérisé en ce qu'**il est conçu pour empêcher la communication de données entre le premier fournisseur de services à distance et le second dispositif local et pour empêcher la communication de données entre le second fournisseur de services à distance et le premier dispositif local.

2. Dispositif de contrôle d'accès selon la revendication 1, le dispositif comprenant une pluralité de machines virtuelles, chacune étant conçue pour gérer la communication de données entre au moins un fournisseur de services à distance respectif et au moins un dispositif local respectif.

3. Dispositif de contrôle d'accès selon l'une quelconque des revendications précédentes, la communication de données locales se faisant par l'intermédiaire d'un réseau local.

4. Dispositif de contrôle d'accès selon l'une quelconque des revendications précédentes, au moins un dispositif local étant un dispositif de mesure.

5. Dispositif de contrôle d'accès selon l'une quelconque des revendications précédentes, le dispositif de contrôle d'accès étant conçu pour recevoir une demande de connexion de l'un des fournisseurs de services à distance, la demande de connexion incluant un identifiant du fournisseur de service à distance, pour rejeter la demande de connexion et, en réponse à la demande de connexion reçue, pour comparer l'identifiant reçu à une liste prédéfinie d'identifiants pour les fournisseurs de services à distance autorisés et, si l'identifiant correspond à l'un des identifiants de la liste, pour identifier une adresse prédéfinie pour le fournisseur de services à distance, l'adresse ayant été préalablement stockée par le dispositif de contrôle d'accès, et pour établir une communication de données avec l'adresse prédéfinie.

6. Dispositif de contrôle d'accès selon l'une quelconque des revendications précédentes, en réponse à une demande de connexion réussie de l'un des fournisseurs de services à distance, le dispositif de contrôle d'accès étant conçu pour établir une communication de données avec l'un des dispositifs locaux associés au fournisseur de services à distance.

7. Dispositif de contrôle d'accès selon l'une quelconque des revendications précédentes, le dispositif de contrôle d'accès étant conçu pour envoyer une demande de connexion à l'un des dispositifs locaux, la demande de connexion incluant un identifiant du dispositif de contrôle d'accès, le dispositif local étant conçu pour rejeter la demande de connexion, et en réponse à la demande de connexion reçue pour comparer l'identifiant reçu à une liste prédéfinie d'identifiants pour les dispositifs de contrôle d'accès autorisés, et, si l'identifiant correspond à l'un des identifiants sur la liste, pour identifier une adresse prédéfinie pour le dispositif de contrôle d'accès, l'adresse ayant été préalablement mémorisée par le dispositif local, et pour établir une communication de données avec l'adresse prédéfinie.

8. Dispositif de contrôle d'accès selon l'une quelconque des revendications précédentes, le dispositif de contrôle d'accès étant conçu pour recevoir une demande de connexion de l'un des dispositifs locaux, la demande de connexion incluant un identifiant du dispositif local, le dispositif de contrôle d'accès étant conçu pour rejeter la demande de connexion, et en réponse à la demande de connexion reçue pour comparer l'identifiant reçu à une liste prédéfinie d'identifiants pour les dispositifs locaux autorisés et, si l'identifiant correspond à l'un des identifiants sur la liste, pour identifier une adresse prédéfinie pour le dispositif local, l'adresse ayant été préalablement mémorisée par le dispositif de contrôle d'accès, et pour établir une communication de données avec l'adresse prédéfinie.

9. Dispositif de contrôle d'accès selon l'une quelconque des revendications précédentes, en réponse à une demande de connexion réussie de l'un des dispositifs locaux, le dispositif de contrôle d'accès étant conçu pour établir une communication de données avec l'un des fournisseurs de services à distance associés au dispositif local.

10. Logiciel qui configure un appareil de traitement de données à usage général pour fonctionner comme un dispositif de contrôle d'accès selon l'une quelconque des revendications précédentes, l'appareil de traitement de données à usage général étant conçu pour :
communiquer des données avec au moins un premier fournisseur de services à distance et un second fournisseur de services à distance par l'intermédiaire d'un réseau de télécommunications étendu ;
communiquer des données avec au moins un premier dispositif local et un second dispositif local par l'intermédiaire d'une communication de données locale ;
faciliter la communication de données entre le premier fournisseur de services à distance et le premier dispositif local ;
faciliter la communication de données entre le second fournisseur de services à distance et le second dispositif local ;
empêcher la communication de données entre le premier fournisseur de services à distance et le second dispositif local ; et
empêcher la communication de données entre le second fournisseur de services à distance et le premier dispositif local.
